# EUROPÄISCHE PATENTANMELDUNG

(11) **EP 2 581 051 A1**
(43) Veröffentlichungstag der Anmeldung: **17.04.2013**
(21) Anmeldenummer: 12008486.8
(22) Anmeldetag: 09.06.2011
(51) Int. Cl.: A61B 17/225, A61H 23/00, A61H 23/04

(54) **Druckwellengerät mit pneumatischem Antrieb**

(30) Priorität: 11.06.2010 DE 202010007860 U
(62) Teilanmeldung aus: 11004690.1
(71) Anmelder: STORZ MEDICAL AG, 8274 Tägerwilen (CH)
(72) Erfinder: Novak, Pavel, 8234 Stetten, SH (CH); Schulz, Manfred, 8274 Tägerwilen/T (CH); Marlinghaus, Ernst H., 8598 Bottighofen/TG (CH); Görner, Georg, 8597 Landschlacht/TG (CH)
(74) Vertreter: Szynka, Dirk

(57) **Zusammenfassung**

Die Erfindung betrifft ein pneumatisches betriebenes Druckwellengerät zur Behandlung des menschlichen oder tierischen Körpers, bei dem ein Kraftsensor vorgesehen ist, über den eine Andruckkraft zumindest teilweise von einer Anlagevorrichtung auf ein Gerätegehäuse übertragen wird.

## Beschreibung

Die vorliegende Erfindung betrifft ein Druckwellengerät zur Behandlung des menschlichen oder tierischen Körpers durch mechanische Druckwellen.

Geräte zur Behandlung mit mechanischen Druckwellen sind an sich bekannt, insbesondere aus dem Bereich der Lithotripsie. Dort werden mit fokussierten mechanischen Druckwellen Körperkonkremente, etwa Steine im Körpergewebe, zertrümmert. Neben der Erzeugung durch elektrische Entladungen im Wasser sind auch Geräte entwickelt worden, die mechanische Druckwellen durch das Aufeinanderprallen eines beschleunigten Schlagteils und eines Prallkörpers erzeugen. Solche Geräte werden sowohl in der Lithotripsie als auch bei anderen Behandlungen biologischer Körpersubstanzen eingesetzt, wobei insbesondere die Behandlung von Muskelerkrankungen und von Erkrankungen im Übergangsbereich zwischen Muskeln und Knochen sowie Anwendungen im dermatologischen Bereich zu nennen sind.

Der vorliegenden Erfindung liegt die Aufgabe zugrunde, ein Druckwellengerät der beschriebenen Art anzugeben, das verbesserte Gebrauchseigenschaften zeigt.

Erfindungsgemäß wird diese Aufgabe durch ein Druckwellengerät mit einem Gehäuse und einer an dem Gehäuse gelagerten Anlagevorrichtung zur Anlage an den Körper gelöst, wobei die Anlagevorrichtung derart über einen Kraftsensor an dem Gehäuse gelagert ist, dass eine Kraftübertragung von der Anlagevorrichtung auf das Gehäuse zumindest teilweise über den Kraftsensor erfolgt.

Das Druckwellengerät kann eine Kombination aus einem Handgerät und einem dieses versorgenden Basisgerät sein, wobei das Basisgerät fest installiert oder bewegbar, also etwa tragbar bzw. mit Rollen verschiebbar sein kann. Der Begriff "Druckwellengerät" meint jedoch auch allein ein beispielsweise batteriebetriebenes unabhängig einsetzbares Handgerät. Der pneumatische Antrieb erfolgt in beiden Fällen über ein Druckgas, etwa mit einem Kompressor komprimierte Luft, dessen Expansion die Druckwelle direkt erzeugen oder zu deren Erzeugung mechanische Komponenten bewegen kann.

Die Grundidee der Erfindung besteht darin, dass für den Betrieb des Druckwellengeräts in Ergänzung zu einer sensorischen Wahrnehmung der Anpresskraft durch einen Benutzer, beispielsweise einen Arzt oder Therapeuten, eine messtechnische Erfassung der Anpresskraft erfolgt. Durch einen erfindungsgemäßen Kraftsensor zwischen dem Gehäuse, über welches der Benutzer die Anpresskraft aufbringt, und der Anlagevorrichtung, welche dann in Abhängigkeit von der Anpresskraft und einer Anlagefläche mit einem bestimmten Anpressdruck am Körper anliegt, kann ein von der Anpresskraft abhängiger und den Anpressdruck bestimmender Messwert ermittelt werden. Eine solche objektivierte Erfassung der Anpresskraft ist vorteilhaft, weil der durch die Anpresskraft bestimmte Anpressdruck die Einkopplung von Druckwellen in den Körper beeinflusst, die mit steigendem Anpressdruck zunimmt. Im Falle eines zu geringen Anpressdrucks kann die Intensität der Druckwellen in dem zu behandelnden Körperteil beispielsweise zu niedrig werden, um noch die gewünschte physiologische Wirkung zu erzielen, wohingegen im Falle eines zu hohen Anpressdrucks Verletzungen, etwa Hautverletzungen wie beispielsweise Hämatome, resultieren können.

Die Anlagevorrichtung ist dabei nicht notwendigerweise allein über den Kraftsensor mit dem Gehäuse verbunden, sondern kann beispielsweise auch in einer Lagervorrichtung des Gehäuses solchermaßen geführt sein, dass eine Relativverschiebung zwischen Anlagevorrichtung und Gehäuse im Wesentlichen nur entlang einer Achse möglich ist (oder unter Außerachtlassung des Kraftsensors wäre). Die Richtung der Relativverschiebung kann dabei etwa so gewählt werden, dass sie im Wesentlichen mit einer Anpressrichtung, in der das Druckwellengerät an den Körper angesetzt wird, und einer Hauptausbreitungsrichtung der Druckwellen, die ein Mittelwert oder Durchschnitt einer Vielzahl von Ausbreitungsrichtungen sein kann, zusammenfällt. In dieser Richtung ist dann vorzugsweise auch eine sensitive Achse des Kraftsensors angeordnet, sodass eine für den Anpressdruck und damit die Behandlung maßgebende Kraftkomponente über den Kraftsensor übertragen und von diesem als Messgröße dem Benutzer bzw. einer Steuereinheit für den Betrieb des Druckwellengeräts zur Verfügung gestellt wird.

Im Detail sind in der Umsetzung eine Vielzahl Möglichkeiten gegeben, um die Lagerung der Anlagevorrichtung am Gehäuse zu realisieren. In Abhängigkeit von der speziellen Lagervorrichtung erfolgt die Kraftübertragung dann nicht notwendigerweise ausschließlich über den Kraftsensor, sondern kann beispielsweise auch durch eine Haftkraft zwischen Anlagevorrichtung und Gehäuse vermittelt sein. Eine solche im Wesentlichen durch Haftreibung bestimmte Haftkraft kann in erster Näherung unabhängig von der Anpresskraft sein und lässt sich, sofern ein Absolutwert ausgegeben werden soll, dann beispielsweise im Zuge einer "offset"-Korrektur bei einer weiteren Verarbeitung des über den Kraftsensor ermittelten Messwerts korrigieren.

Bevorzugte Ausgestaltungen der Erfindung sind in den abhängigen Ansprüchen angegeben. Im Folgenden wird wie in der gesamten Offenbarung nicht im Einzelnen zwischen der Beschreibung des Druckwellengeräts und dem Verwendungsaspekt der Erfindung unterschieden, die Offenbarung ist implizit im Hinblick auf beide Kategorien zu verstehen.

Eine Ausgestaltung der Erfindung sieht vor, dass der Kraftsensor die Kraftübertragung piezoelektrisch oder piezoresistiv misst. Mit piezoelektrischen Sensoren wird eine durch kraftinduzierte Verformung hervorgerufene Ladungstrennung in einem piezoelektrischen Material mit beispielsweise einem Ladungsverstärker ausgewertet und dann als zur Kraft proportionale Messgröße zur Verfügung gestellt. Dabei sind piezoelektrische Sensoren insbesondere für eine gute Linearität bekannt und zeichnen sich ferner durch Robustheit und Unempfindlichkeit gegen elektromagnetische Verunreinigung aus.

Bei einem piezoresistiven Drucksensor bewirkt eine Krafteinwirkung eine Verformung und damit Widerstandsänderung eines Dehnungsmessstreifens infolge dessen Dehnung beziehungsweise Stauchung. Der Dehnungsmessstreifen kann beispielsweise als Metall aufgebracht sein, etwa auf siliziumhaltiges Material wie Siliziumnitrid, oder durch Dotierung eingebracht werden, etwa in Silizium eindiffundiert, wobei das Substrat auch als Membran entsprechend dünn ausgeführt sein kann. Alternativ zu einem Siliziumsubstrat kann ein Dehnungsmessstreifen auch auf einem Kunststoffträger realisiert werden, wobei ein typischerweise mäanderförmiges Messgitter beispielsweise durch entsprechendes Kaschieren und Ätzen einer Metallfolie hergestellt sein kann.

Eine piezoelektrische beziehungsweise piezoresistive Kraftmessung kann dann beispielsweise derart realisiert sein, dass ein elastisch verformbares Element, etwa ein Elastomerblock, zwischen der Anlagevorrichtung und dem Gehäuse vorgesehen ist. Wird dann etwa mit einem Dehnungsmessstreifen die Verformung des Elements unter Krafteinwirkung bestimmt, kann bei Kenntnis einer entsprechenden Materialkonstante die über das Element übertragene Kraft ermittelt werden. Inwiefern bei der Messung eine makroskopische Relativverschiebung zwischen Anlagevorrichtung und Gehäuse erfolgt, hängt auch von dem Elastizitätsmodul des elastischen Elements ab. Wenngleich hier keine scharfe Grenze angegeben werden kann, wird vorliegend bei einem Elastizitätsmodul von deutlich unterhalb 1 GPa von einer Verformung des Elements und damit Relativverschiebung gesprochen. Die Verformung des elastischen Elements kann dabei beispielsweise in Richtung der Relativverschiebung oder auch quer zu dieser gemessen werden, sodass im letztgenannten Fall etwa eine bei Kompression in Richtung der Relativverschiebung quer zu dieser erfolgende Expansion ermittelt wird, also eine Verdrängung des elastischen Elements.

Es kann auch ein Fluidkissen als Kopplungselement zwischen der Anlagevorrichtung und dem Gehäuse vorgesehen sein, wobei die Kraftmessung dann beispielsweise über eine in dem Fluid ermittelte Druckänderung erfolgen kann. Das Fluidkissen wird in Abhängigkeit von seiner Form, den Verformungseigenschaften einer das Fluid umschließenden Hülle beziehungsweise der Viskosität des Fluids selbst mit mehr oder weniger Verformung auf die Krafteinwirkung reagieren, kann also so gestaltetet sein, dass die Messung bei einer Relativverschiebung zwischen Anlagevorrichtung und Gehäuse oder im Wesentlichen ohne eine solche erfolgt.

In weiterer Ausgestaltung erfolgt die Kraftmessung weglos, also im Wesentlichen ohne Relativverschiebung zwischen Anlagevorrichtung und Gehäuse. So reagiert beispielsweise ein piezoelektrischer Sensor aus etwa einer piezoelektrischen Keramik oder einem einkristallinen Material aufgrund der hohen Elastizitätsmoduli dieser Materialien auf eine Kraft praktisch ohne Verformung. Erfolgt die Kraftübertragung dann direkt über den Sensor, ist dessen geringe Verformung maßgebend für die Relativverschiebung, sodass die Kraftmessung im Wesentlichen ohne Relativverschiebung zwischen Anlagevorrichtung und Gehäuse erfolgt. Für einen Benutzer ergibt sich dadurch bei der Handhabung des Druckwellengeräts in seiner eigenen sensorischen Wahrnehmung kein Unterschied zu den ihm bekannten Geräten.

Das gilt auch im Falle einer piezoresistiven Messung mit entweder einem Dehnungsmessstreifen, über dessen Membran mit hohem Elastizitätsmodul die Kraftübertagung direkt erfolgt, oder einem Kunststoffträger-basierten Dehnungsmessstreifen, der auf einem Kopplungselement mit entsprechend hohem Elastizitätsmodul vorgesehen ist, etwa auf einem Metallblock.

Wenngleich sowohl bei der direkten Kraftübertragung über einen piezoelektrischen Sensor als auch bei den eben dargestellten Möglichkeiten zur piezoresistiven Messung eine Relativverschiebung im Bereich von Mikrometern und darunter erfolgen kann, wird eine solche Relativverschiebung von in dieser Reihenfolge zunehmend bevorzugt weniger als 50 µm, 30 µm, 10 µm, 1 µm in der gesamten Offenbarung als "keine Relativverschiebung" bezeichnet und die Messung als "weglos".

Bei einer anderen Ausführungsform misst der Kraftsensor eine Relativverschiebung zwischen der Anlagevorrichtung und dem Gehäuse optisch, elektrisch, elektromagnetisch oder magnetisch. Bei dieser Ausführungsform bewirkt die Anpresskraft also eine Relativverschiebung, die optisch, etwa durch eine laserbasierte Entfernungsmessung, oder elektrisch, beispielsweise durch eine kapazitive Abstandsmessung, gemessen werden kann. Ferner kann die Messung auch elektromagnetisch, etwa über Induktion in einer Spule durch das Feld einer mit Wechselstrom gespeisten weiteren Spule, oder auch magnetisch, beispielsweise über einen Hall-Sensor in Verbindung mit einem Permanentmagneten, erfolgen.

Indem die Relativverschiebung dabei beispielsweise mit einer elastisch verformbaren Lagerung realisiert wird, ergibt sich zusammen mit dem typischerweise wesentlich unterhalb 1 GPa liegenden Elastizitätsmodul der Lagerung ein entsprechender Kraftwert. Eine solche elastische Lagerung kann beispielsweise mit einem elastisch verformbaren Element zwischen Gehäuse und Anlagevorrichtung ausgeführt werden, also etwa einem Elastomerblock oder Elastomerring, insbesondere einem O-Ring. Ferner kann als elastisches Element auch ein oben genanntes und entsprechend verformbar, also eine Relativverschiebung erlaubend, gestaltetes Fluidkissen vorgesehen sein.

In weiterer Ausgestaltung ist die Relativverschiebung zwischen der Anlagevorrichtung und dem Gehäuse durch einen Federkörper kraftbeaufschlagt. Der Federkörper reagiert dabei, gegebenenfalls in einer ausgezeichneten Achse, auf eine Krafteinwirkung mit einer Verformung und kehrt nach Wegnahme der Krafteinwirkung in den Ausgangszustand zurück, sofern die Kraft betragsmäßig eine gewisse Grenze nicht überschreitet. Als solches Federelement kann beispielsweise eine Schraubenfeder, insbesondere eine Schraubendruckfeder, zwischen der Anlagevorrichtung und dem Gehäuse vorgesehen und mit ihrer Schraubenachse dann im Wesentlichen parallel zur Anpressrichtung ausgerichtet werden. Die Relativverschiebung einer beispielsweise in der oben beschriebenen Weise verschiebbar am Gehäuse gelagerten Anlagevorrichtung wird dann durch die Schraubendruckfeder kraftbeaufschlagt, wobei die Kraft zumindest innerhalb eines gewissen Bereichs vorzugsweise direkt proportional zu der Relativverschiebung ist. Die Verschiebbarkeit der Anlagevorrichtung vom Gehäuse weg kann dabei durch einen Anschlag begrenzt sein, der die Schraubendruckfeder gegebenenfalls schon teilweise komprimiert und die Anlagevorrichtung somit in Position hält, auch wenn diese noch nicht an den Körper gepresst wird. Die Schraubenfeder kann beispielsweise aus Kunststoff oder einem metallischen Werkstoff hergestellt sein, etwa aus Draht gewickelt.

Durch die federbeaufschlagte Relativverschiebung zwischen Anlagevorrichtung und Gehäuse wird insbesondere der Anstieg des Anpressdrucks beim Ansetzen nicht allein von der Konsistenz des zu behandelnden Körperteils bestimmt, sondern beispielsweise auch bei verformungsarm reagierenden Körperregionen mit einer der Relativverschiebung entsprechenden Verzögerung aufgebaut. Dem Benutzer steht somit eine Übersetzung, deren Übersetzungsverhältnis mit abnehmender Federkonstante zunimmt, zwischen seiner Anpressbewegung und dem Anpressdruck zur Verfügung, sodass der Anpressdruck genauer eingestellt und die objektivierte Erfassung über den Kraftsensor besonders vorteilhaft genutzt werden kann.

Nach einer weiteren Ausführungsform wird die Erzeugung einer Druckwelle freigegeben oder ausgelöst, wenn ein über den Kraftsensor ermittelter Wert einen Minimalwert überschreitet. Für den Benutzer ist diese Funktion vorteilhaft, weil er entweder erst bei Erreichen des gewünschten Minimalwerts die Druckwelle auslösen kann oder die Auslösung der Druckwelle dann sogar nicht mehr selbst betätigen muss. Sofern die Druckwellenauslösung bei einem bestimmten Anpressdruck erfolgen soll, wird nicht nur ein Handgriff eingespart, sondern beispielsweise auch eine ansonsten komplexe Auge-Hand-Koordination erleichtert. Darüber hinaus ermöglicht diese Funktion auch auf einfache Weise eine Behandlung mit wiederholt ausgelösten Druckwellen bei nahezu identischem Anpressdruck, wodurch die Effektivität der Behandlung erhöht und potenzielle Verletzungen als Folge eines zu hohen Anpressdrucks besser vermieden werden können. Ein entsprechender Minimalwert kann bei bevorzugten Ausführungen dabei insbesondere auch einstellbar sein und etwa an das spezifische Behandlungsziel angepasst werden.

Bei einer weiteren Ausführungsform ist das Auslösen der Erzeugung der Druckwelle blockiert, wenn ein über den Kraftsensor ermittelter Wert einen Maximalwert überschreitet. Bei dieser Ausführungsform ist also ein Sicherungsmechanismus vorgesehen, der die Erzeugung der Druckwelle blockiert, wenn die Einkopplung der Druckwelle in den Körper aufgrund einer zu hohen Anpresskraft des Druckwellengeräts eine unerwünscht hohe Intensität der Druckwellenbehandlung oder Hautreizung zur Folge hätte. Der Maximalwert kann dabei insbesondere auch einstellbar sein, und etwa bei der Behandlung sensitiver Körperregionen herabgesetzt werden.

In weiterer Ausgestaltung des Druckwellengeräts ist ein über den Kraftsensor ermittelter Wert auf einer optischen Anzeige ablesbar. Der optischen Anzeige kann dabei ein analoges oder digitales Signal des Kraftsensors zugeführt werden, das gegebenenfalls in einer Auswerteeinheit zuvor entsprechend aufbereitet, also beispielsweise digitalisiert oder verstärkt und insbesondere auch offset-korrigiert, werden kann. Dem Benutzer kann dann beispielsweise ein im Wesentlichen der Anpresskraft entsprechender Wert, ein dem Anpressdruck, also der Anpresskraft normiert auf die Anlagefläche, entsprechender Wert oder auch eine dimensionslose Größe zur Verfügung gestellt werden.

Die optische Anzeige kann den Wert beispielsweise über digitale Ziffern oder einen Zeiger mit entsprechender Skala darstellen und allein am Handgerät bzw. im Falle eines Druckwellengeräts mit versorgendem Basisgerät auch allein oder zusätzlich an diesem angebracht sein.

Es ist dem Benutzer durch die optische Anzeige einfacher möglich, beispielsweise in Abhängigkeit von dem spezifischen Behandlungszweck bzw. dem zu behandelnden Körperteil, die zur optimalen Druckwelleneinkopplung notwendige Anpresskraft aufzubringen. Ferner kann die Anpresskraft auch leichter über den Behandlungsverlauf konstant gehalten bzw. gezielt verändert werden. Die optische Anzeige kann dabei in Ergänzung zu einer selbsttätig Druckwellen auslösenden oder deren Auslösung blockierender bzw. auch freigebenden Steuereinheit vorliegen, stellt anderenfalls jedoch auch für sich allein eine Umsetzung des Erfindungsgedankens dar, indem einem Benutzer Information für die Bedienung des Druckwellengeräts zur Verfügung gestellt wird.

In weiterer Ausgestaltung ist ein akustischer Signalgeber vorgesehen, der dazu ausgelegt ist, bei einem bestimmten über den Kraftsensor ermittelten Wert ein akustisches Signal auszugeben. Hierbei können auch mehrere voneinander verschiedene akustische Signale für verschiedene Messwerte hinterlegt sein, sodass beispielsweise ein erster Signalton das Erreichen eines gewünschten Anpressdrucks signalisiert und ein Warnton das Überschreiten eines kritischen Anpressdrucks. Ferner besteht auch die Möglichkeit, eine Veränderung des Anpressdrucks durch Veränderung der Lautstärke anzuzeigen, sodass beispielsweise eine mit steigendem Anpressdruck zunehmende Lautstärke eine intuitive Bedienung ermöglichen kann. Hierbei ist wiederum eine beliebige Kombination mit automatischer Steuerung und auch optischer Anzeige möglich.

Bei einer weiteren Ausführungsform ist das Druckwellengerät ausgelegt für eine Kraftmessung des Kraftsensors nach der Erzeugung der Druckwelle, die ein Ansprechverhalten des Körpers auf die Einkopplung der Druckwelle erfasst. Ändert sich durch die Druckwellenbehandlung also beispielsweise die Gewebe- bzw. Muskelsteifigkeit, kann eine solche Veränderung dann mit einer auf die Erzeugung der Druckwelle folgenden Kraftmessung erfasst werden, weil die veränderte Steifigkeit ein verändertes Vibrationsverhalten des behandelten Körperteils zur Folge hat und dieses mit dem Sensor erfasst werden kann. Es kann also beispielsweise über eine mit dem Kraftsensor vorgenommene Eigenfrequenzmessung des behandelten Körperteils bzw. Körperbereichs eine Ab- oder Zunahme von dessen Steifigkeit beobachtet werden. Da die Kraftmessung zur Bestimmung des Ansprechverhaltens des Körpers mit dem selben Kraftsensor erfolgt, der auch den Anpressdruck misst, ist die Kombination der beiden Messungen auf besonders einfache und ökonomisch vorteilhafte Weise möglich.

In weiterer Ausgestaltung ist dabei ein Wert der Kraftmessung einer Regeleinheit zuführbar, welche Regeleinheit dazu ausgelegt ist, den Wert als Regelgröße für eine Einstellung einer nachfolgenden Druckwelle zu verwenden, sodass die Einstellung an das Ansprechverhalten des Körpers angepasst ist. Das Ansprechverhalten des Körpers kann beispielsweise durch die Steifigkeit von Gewebe bzw. Muskeln oder eine Beweglichkeit einzelner Körperteile bzw. Knochen charakterisiert sein. In Abhängigkeit von dem spezifischen Behandlungsziel wird eine entsprechende Größe dann beispielsweise über mehrere Druckwellen hinweg ausgewertet, sodass etwa die Intensität der Druckwellen erhöht werden kann, wenn sich keine Veränderung zeigt. Die Intensität kann andernfalls etwa auch gesenkt bzw. die Behandlung vollständig abgebrochen werden, wenn die entsprechende Größe nach einer Phase der Veränderung im Wesentlichen unverändert bleibt.

Die Erfindung bezieht sich auch auf ein Druckwellengerät, bei dem eine Anpressvorrichtung als Bestandteil des Gehäuses zum Anpressen eines zu behandelnden Körperteils an die Anlagevorrichtung ausgelegt ist. In Ergänzung zum Anpressen des Druckwellengeräts kann also auch der zu behandelnde Körperteil mit einer für diesen Zweck vorgesehenen mechanischen Anpressvorrichtung an das Druckwellengerät gepresst werden. Da die Anpressvorrichtung Bestandteil des Gehäuses ist, erfolgt dabei eine Übertragung einer für den Anpressdruck maßgeblichen Kraftkomponente von der Anlagevorrichtung auf das Gehäuse und somit über den Kraftsensor. Sofern im Kontext dieser Offenbarung also eine Bewegung des Druckwellengeräts zu dem Körperteil hin beschrieben wird, ist die dargestellte Funktionalität ebenso auf eine Bewegung des Körperteils zu dem Druckwellengerät hin zu lesen.

Bei einer weiteren Ausführungsform weist das Druckwellengerät ein Schlagteil auf, das durch den pneumatischen Antrieb bewegbar und für einen Aufprall zur Erzeugung der Druckwelle ausgelegt ist. Das Schlagteil kann beispielsweise ein in einer linearen Führungsvorrichtung geführtes Projektil sein, das in der Führungsvorrichtung durch eine Druckgasexpansion beschleunigt wird. Der pneumatische Antrieb kann jedoch beispielsweise auch eine Rotationsbewegung einer Achse antreiben, mit der das Schlagteil verbunden ist und somit auf einer Kreisbahn bewegt wird. Zur Erzeugung der Druckwelle kann das Schlagteil direkt auf den Körper oder auch auf eine weitere Vorrichtung prallen.

Eine solche Vorrichtung ist in weiterer Ausgestaltung ein Prallkörper zur Erzeugung der Druckwelle infolge eines Druckpulses des pneumatischen Antriebs. Eine Druckwelle kann dabei einerseits in der eben beschriebenen Weise durch den Aufprall eines Schlagteils auf den Prallkörper erzeugt werden, andernfalls aber auch alleine durch die Wechselwirkung eines Druckpulses, also der Expansion des Druckgases, mit dem Prallkörper entstehen, etwa indem ein Druckpuls den Prallkörper aus einer Ruheposition bewegt.

Bei einer weiteren Ausführungsform ist zur Führung eines solchen Druckpulses des pneumatischen Antriebs ein Führungsrohr vorgesehen, und weiter bevorzugt dabei mit einem Druckpuls ein Schlagteil in dem Führungsrohr bewegbar, um am Ende einer Bewegung durch das Führungsrohr die Druckwelle durch einen Aufprall auf den Prallkörper zu erzeugen.

Bei einer Ausgestaltung der Erfindung meint "Anlagevorrichtung" den Prallkörper, sodass die Kraftübertragung von dem Prallkörper auf das Gehäuse zumindest teilweise über den Kraftsensor erfolgt. Sofern bei dieser Ausgestaltung also ein Führungsrohr vorhanden ist, zählt es zum Gehäuse und ist im Rahmen üblicher Montagetechniken fest mit diesem verbunden.

Bei einer anderen Ausführungsform hingegen ist das Führungsrohr die an dem Gehäuse gelagerte Anlagevorrichtung, sodass die Kraftübertragung von dem Führungsrohr auf das Gehäuse zumindest teilweise über den Kraftsensor erfolgt. Sofern bei dieser Ausführungsform ein Prallkörper vorhanden ist, bildet dieser dann zusammen mit dem Führungsrohr die Anlagevorrichtung und ist der beim Betrieb am Körper anliegende Teil davon. Dabei kann der Prallkörper insbesondere auch elastisch am Führungsrohr gelagert sein, beispielsweise mit Elastomerringen, etwa O-Ringen.

Die Erfindung bezieht sich auch auf die Verwendung eines Druckwellengeräts zum Anpressen an einen menschlichen oder tierischen Körper und Messen einer Anpresskraft. Ein erfindungsgemäßes Druckwellengerät kann also beispielsweise zur Objektivierung des Schmerzempfindens bzw. zum Auffinden von Triggerpunkten auf eine entsprechende Körperregion gesetzt und mit steigender Kraft angedrückt werden, wobei eine über den Kraftsensor erfolgende Messung des Anpressdrucks eine reproduzierbare Quantifizierung der lokalen Druckschmerzschwelle ermöglicht (F-Meter-Funktion). Eine solche Verwendung kann auch unabhängig von einer Druckwellenbehandlung erfolgen, sodass dem Benutzer vorteilhafterweise mit nur einem Gerät zwei verschiedene Anwendungsgebiete eröffnet sind. Durch die Kombination der F-Meter-Funktion mit einer Druckwellenbehandlung können jedoch auch beispielsweise vor der Behandlung die für eine Behandlung relevanten Stellen identifiziert werden bzw. im Anschluss an eine Behandlung der Erfolg dieser überprüft werden.

Im Folgenden wird die Erfindung anhand von Ausführungsbeispielen näher erläutert, wobei die einzelnen Merkmale auch in anderen Kombinationen erfindungswesentlich sein können und sich implizit auf alle Kategorien der Erfindung beziehen.
- Figur 1: zeigt ein Druckwellengerät mit federbeaufschlagter Relativverschiebung zwischen Führungsrohr und Gehäuse.
- Figur 2: zeigt eine piezoresistive Kraftmessung zwischen Führungsrohr und Gehäuse.
- Figur 3: zeigt eine federbeaufschlagte Relativverschiebung zwischen Prallkörper und Führungsrohr.

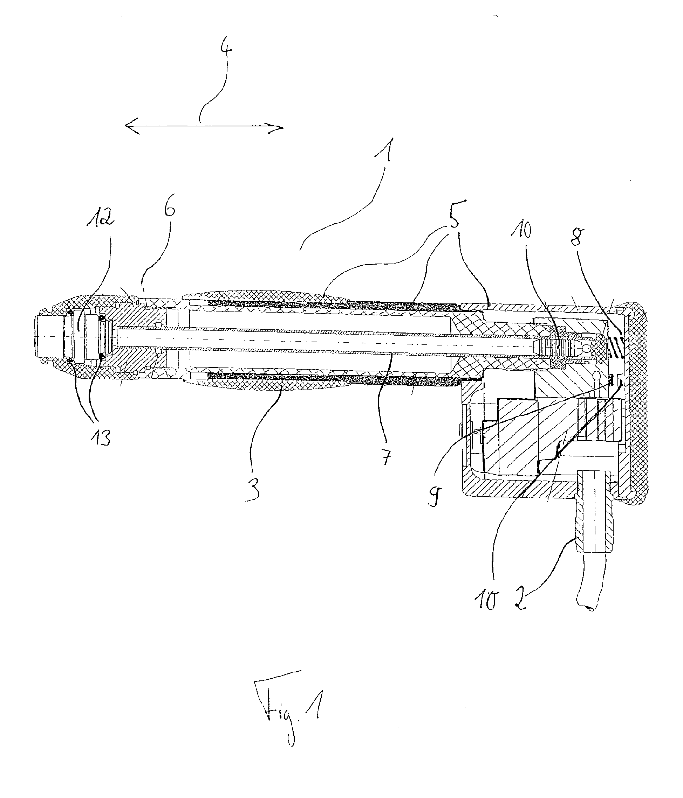

Figur 1 zeigt ein Handstück eines Druckwellengeräts 1 zur Behandlung des menschlichen oder tierischen Körpers mit mechanischen Druckwellen, welches von einem nicht dargestellten mit einem Kompressor ausgestatteten Basisgerät über einen Versorgungsanschluss 2 mit Druckgas versorgt wird. Das Handgerät kann beispielsweise von einem behandelnden Arzt an der Grifffläche 3 gegriffen und mit dem linken Ende an den Körper angesetzt und in der Richtung 4 angedrückt werden. Die Grifffläche 3 bildet zusammen mit weiteren Komponenten, deren Relativposition zueinander im montierten Zustand festgelegt ist, das Gehäuse 5. In einem Schaft des Gehäuses 5 ist eine Anlagevorrichtung 6 so gelagert, dass eine Relativverschiebung zwischen Anlagevorrichtung 6 und Gehäuse 5 entlang der Achse 4 möglich ist. In der Anlagevorrichtung 6 ist ein Führungsrohr 7 gehalten und dabei axial und konzentrisch angeordnet. Die Relativverschiebung zwischen Gehäuse 5 und Anlagevorrichtung 6 mit Führungsrohr 7 ist durch den Federkörper 8 kraftbeaufschlagt, sodass für ein zum Gehäuse 5 hin, also in der Abbildung nach rechts, Schieben des Führungsrohrs 7 eine mit der verschobenen Distanz zunehmende Kraft erforderlich ist. Über Distanzmessungen mit einem Permanentmagneten 9 und einem Hallsensor 10 kann die Relativverschiebung zwischen der Anlagevorrichtung 6 und dem Gehäuse 5 bestimmt werden. Aus dieser ergibt sich dann in Abhängigkeit von der Federkonstanten und unter Berücksichtigung einer Vor-Kompression ein entsprechender Kraftwert.

Die bei einem Ansetzen des in der Darstellung linken Endes des Druckwellengeräts 1 und Anpressen entlang der Achse 4 erfolgende Relativverschiebung zwischen Führungsrohr 7 und Gehäuse 5 liefert somit einen Kraftwert, der im Wesentlichen der Anpresskraft entspricht. Die Feder 8 wird durch die vom Benutzer aufgebrachte Kraft zwischen der Anlagevorrichtung 6 und dem Gehäuse 5 komprimiert und bewirkt ihrerseits eine entsprechende Gegenkraft, mit der die Anlagevorrichtung 6 angedrückt wird. Aufgrund von Reibungskräften in der Lagerung zwischen Gehäuse 5 und Führungsrohr 7 wird jedoch nicht notwendigerweise die gesamte vom Benutzer aufgebrachte Kraft allein über die Feder 8 auf die Anlagevorrichtung 6 übertragen. In dem Führungsrohr 7 ist ein Schlagteil 11 geführt, das durch einen Druckpuls des Druckgases entlang der Achse 4 nach links beschleunigt werden kann und in dieser Bewegung durch einen Prallkörper 12 begrenzt ist. Das Schlagteil 11 wird also von einem proximalen (rechten) zu einem distalen (linken) Ende des Führungsrohres 7 beschleunigt und erzeugt dann durch den Zusammenprall mit einem proximalen Ende des Prallkörpers 12 eine Druckwelle, die über das distale Ende des Prallkörpers 12 in den Körper eingekoppelt wird. Der Prallkörper 12 ist über zwei O-Ringe 13 elastisch gegenüber der Anlagevorrichtung 6 gelagert.

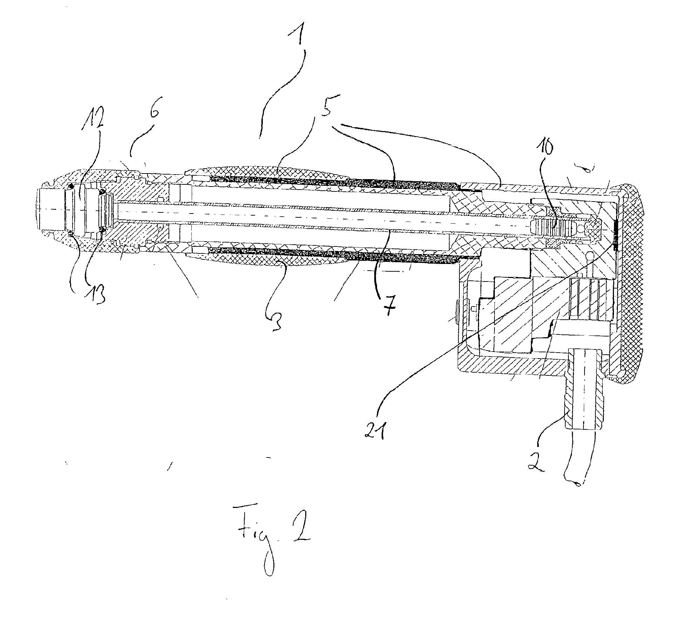

Bei dem in Figur 2 dargestellten Druckwellengerät sind Teile mit zu Figur 1 identischer Funktion mit den selben Bezugszeichen versehen. Der Prallkörper 12 bildet wiederum zusammen mit dem Führungsrohr 7 die Anlagevorrichtung 6, welche an dem Gehäuse 5 gelagert ist. Wie auch in Figur 1 ist die Anlagevorrichtung 6 dabei in einem axialen Schaft des Gehäuses geführt, wobei zwischen Schaft und Anlagevorrichtung 6 keine weitere kraftvermittelnde Verbindung vorgesehen ist, also im Wesentlichen die Haftkraft dominiert. Im Gegensatz zu Figur 1 ist bei diesem Ausführungsbeispiel dennoch keine Relativverschiebung der Anlagevorrichtung 6 zum Gehäuse 5 möglich, weil zwischen Anlagevorrichtung 6 und Gehäuse 5 kein Federelement 8, sondern ein piezoresistiver Sensor 21 vorgesehen ist.

Der piezoresistive Sensor 21 misst eine Druckänderung, welche in Abhängigkeit von seiner Fläche einer Krafteinwirkung entspricht, über eine elektrische Widerstandsänderung des piezoresistiven Materials. Hierbei kommt es zwar zu einer Verformung des piezoresistiven Materials, allerdings liegt diese größenordnungsmäßig im Bereich von Mikrometern, sodass bei makroskopischer Betrachtung im Wesentlichen keine Relativverschiebung zwischen der Anlagevorrichtung 6 und dem Gehäuse 5 erfolgt.

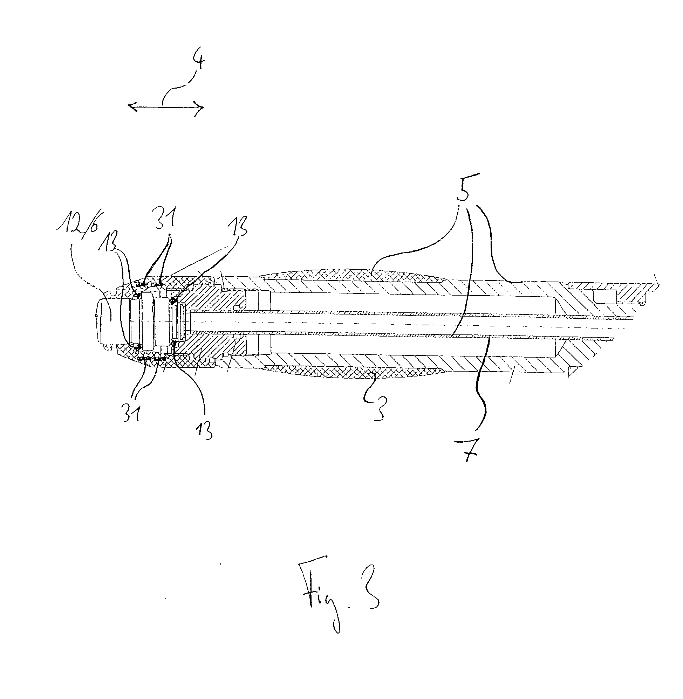

Figur 3 zeigt das Führungsrohr 6 und den Prallkörper 12 eines Druckwellengeräts 1, das wie in der zu Figur 1 beschriebenen Weise Druckwellen durch den Aufprall eines Schlagteils 11 auf den Prallkörper 12 erzeugt. In diesem Fall ist jedoch das Führungsrohr 7 nicht beweglich am Gehäuse 5 gelagert, sondern ist als Teil des Gehäuses 5 in seiner Position zu diesem festgelegt. Die am Gehäuse 5 gelagerte Anlagevorrichtung ist in diesem Fall der Prallkörper 12, der durch die Elastomerringe 13 gegenüber dem Gehäuse beweglich gelagert ist. Beim Anpressen des Druckwellengeräts 1 an den Körper wird der Prallkörper 12 zum Gehäuse 5 hin verschoben, wobei diese Verschiebung mit zwei Differenzialspulen 31 gemessen wird. Bei der Verschiebung wird der Proximale der beiden Elastomerringe 13 komprimiert, wobei die Kompression proportional zur wirkenden Kraft ist, die dann aus der Verschiebung und einem Elastizitätsmodul des O-Rings 13 bestimmt werden kann.

Bei dieser Ausführungsform kann am Führungsrohr 7 eine Anpressvorrichtung vorgesehen werden, mit der dann der zu behandelnde Körperteil umgriffen und zum Prallkörper 12 hin gepresst werden kann. Da die Kraftmessung dabei zwischen der Anpress- und der Anlagevorrichtung erfolgt, wird ein für den Anpressdruck maßgebender Wert erfasst.

Im Vergleich zu den in den Figuren 1 und 2 gezeigten Ausführungsbeispielen kann der Elastizitätsmodul des O-Rings 13 gegebenenfalls kleiner eingestellt werden, um eine gut messbare Relativverschiebung zu erlauben. Sofern andererseits auch in den Figuren 1 und 2 eine nicht vernachlässigbare Relativverschiebung erfolgt, kann diese beispielsweise bei einer Auswertung berücksichtigt und der gemessene Kraftwert entsprechend korrigiert werden.

## Patentansprüche

1. Druckwellengerät (1) zur Behandlung des menschlichen oder tierischen Körpers,
mit einem pneumatischen Antrieb zur Erzeugung einer Druckwelle,
mit einem Gehäuse (5) und einer Anlagevorrichtung (6) zur Anlage an den Körper,
wobei die Anlagevorrichtung (6) an dem Gehäuse (5) gelagert ist,
**dadurch gekennzeichnet, dass**
die Anlagevorrichtung (6) derart über einen Kraftsensor an dem Gehäuse (5) gelagert ist, dass eine Kraftübertragung von der Anlagevorrichtung (6) auf das Gehäuse (5) zumindest teilweise über den Kraftsensor erfolgt.

2. Druckwellengerät (1) nach Anspruch 1, bei dem der Kraftsensor (21) die Kraftübertragung piezoelektrisch oder piezoresistiv misst.

3. Druckwellengerät (1) nach Anspruch 1, bei dem der Kraftsensor eine Relativverschiebung zwischen der Anlagevorrichtung (6) und dem Gehäuse (5) optisch, elektrisch, elektromagnetisch oder magnetisch misst.

4. Druckwellengerät (1) nach Anspruch 3, bei dem die Relativverschiebung zwischen der Anlagevorrichtung (6) und dem Gehäuse (5) durch einen Federkörper (8) zusätzlich kraftbeaufschlagt ist

5. Druckwellengerät (1) nach einem der vorstehenden Ansprüche, bei dem die Erzeugung einer Druckwelle freigegeben oder ausgelöst wird, wenn ein über den Kraftsensor ermittelter Wert einen Minimalwert überschreitet.

6. Druckwellengerät (1) nach einem der vorstehenden Ansprüche, bei dem das Auslösen der Erzeugung der Druckwelle blockiert ist, wenn ein über den Kraftsensor ermittelter Wert einen Maximalwert überschreitet.

7. Druckwellengerät (1) nach einem der vorstehenden Ansprüche, bei dem ein über den Kraftsensor ermittelter Wert auf einer optischen Anzeige ablesbar ist.

8. Druckwellengerät (1) nach einem der vorstehenden Ansprüche mit einem akustischen Signalgeber, der dazu ausgelegt ist, bei einem bestimmten über den Kraftsensor ermittelten Wert ein akustisches Signal auszugeben.

9. Druckwellengerät (1) nach einem der vorstehenden Ansprüche, ausgelegt für eine Kraftmessung des Kraftsensors nach der Erzeugung der Druckwelle, die ein Ansprechverhalten des Körpers auf die Einkopplung der Druckwelle erfasst.

10. Druckwellengerät (1) nach Anspruch 9, bei dem ein Wert der Kraftmessung einer Regeleinheit zuführbar ist, welche Regeleinheit dazu ausgelegt ist, den Wert als Regelgröße für eine Einstellung einer nachfolgenden Druckwelle zu verwenden, sodass die Einstellung an das Ansprechverhalten des Körpers angepasst ist.

11. Druckwellengerät (1) nach einem der vorstehenden Ansprüche, bei dem eine Anpressvorrichtung als Bestandteil des Gehäuses (5) zum Anpressen eines zu behandelnden Körperteils an die Anlagevorrichtung (6) ausgelegt ist.

12. Druckwellengerät (1) nach einem der vorstehendem Ansprüche mit einem Schlagteil (10), das durch den pneumatischen Antrieb bewegbar und zu einem Aufprall zur Erzeugung der Druckwelle ausgelegt ist.

13. Druckwellengerät (1) nach einem der vorstehenden Ansprüche mit einem Prallkörper (12) zur Erzeugung der Druckwelle infolge eines Druckpulses des pneumatischen Antriebs.

14. Druckwellengerät (1) nach Anspruch 13, bei welchem der Prallkörper (12) die an dem Gehäuse (5) gelagerte Anlagevorrichtung (6) ist, sodass die Kraftübertragung von dem Prallkörper (12) auf das Gehäuse (5) zumindest teilweise über den Kraftsensor erfolgt.

15. Druckwellengerät (1) nach einem der vorstehenden Ansprüche, bei welchem ein Führungsrohr (7), welches zur Führung eines Druckpulses des pneumatischen Antriebs ausgelegt ist, die an dem Gehäuse (5) gelagerte Anlagevorrichtung (6) ist, sodass die Kraftübertragung von dem Führungsrohr (7) auf das Gehäuse (5) zumindest teilweise über den Kraftsensor erfolgt.
